(19) Europäisches Patentamt
European Patent Office
Office européen des brevets

(11) Veröffentlichungsnummer: **0 203 418 B1**

(12) # EUROPÄISCHE PATENTSCHRIFT

(45) Veröffentlichungstag der Patentschrift: **11.07.90**

(51) Int. Cl.[5]: **A61K 7/075,** A61K 7/50, C11D 1/32

(21) Anmeldenummer: **86106121.6**

(22) Anmeldetag: **05.05.86**

(54) **Oligopeptid-Derivate, deren Herstellung und deren Verwendung als hautfreundliche Tenside.**

(30) Priorität: **13.05.85 DE 3517205**

(43) Veröffentlichungstag der Anmeldung:
**03.12.86 Patentblatt 86/49**

(45) Bekanntmachung des Hinweises auf die Patenterteilung:
**11.07.90 Patentblatt 90/28**

(84) Benannte Vertragsstaaten:
**AT BE CH DE FR GB IT LI NL SE**

(56) Entgegenhaltungen:
**EP-A- 0 028 779**
**EP-A- 0 050 686**

**CHEMICAL ABSTRACTS, Band 92, Nr. 24, Juni 1980, Seite 321, Nr. 203420j, Columbus, Ohio, US; & JP - A - 79 163 828**
**CHEMICAL ABSTRACTS, Band 93, Nr. 6, August 1980, Seite 450, Nr. 53793n, Columbus, Ohio, US; & JP - A - 80 28 947**

(73) Patentinhaber: **Henkel Kommanditgesellschaft auf Aktien, Postfach 1100 Henkelstrasse 67, D-4000 Düsseldorf-Holthausen(DE)**

(72) Erfinder: **Möller, Hinrich, Dr., Schumannstrasse 11, D-4019 Monheim(DE)**
Erfinder: **Zeidler, Ulrich, Dr., Heinrich-Lersch-Strasse 19, D-4000 Düsseldorf 13(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Gegenstand der Erfindung sind neue Oligopeptid-Derivate, die durch Umsetzung von Alkyl- und Alkenylbernsteinsäureanhydriden mit Proteinhydrolysaten erhältlich sind.

Es ist bekannt, daß die Hautverträglichkeit von Tensiden und tensidhaltigen Zubereitungen durch Zusatz von wasserlöslichen Eiweißstoffen oder Eiweißabbauprodukten merklich verbessert werden kann. Man hat auch bereits Eiweißabbauprodukte in Tenside eingebaut, z.B. durch Acylierung von Protein-Hydrolysaten mit Fettsäurechloriden oder durch Kondensation von Fettsäuremethylestern mit Proteinhydrolysaten. Acylierte Oligopeptide dieser Art sind z.B. unter den Handelsbezeichnungen "Lamepon(R)" und "Maypon(R)" als hautfreundliche Tenside bekannt geworden.

Aus JP-A-79/163 828, referiert in Chem. Abstr. 92 (1980), 203420j war bekannt, daß succinylierte Proteinhydrolysate eine hautfeuchthaltende Wirkung haben und sich als Komponenten in befeuchtenden Hautpflegemitteln eignen.

Die bekannten Fettsäuren-Proteinhydrolysat-Kondensationsprodukte weisen aber anwendungstechnische Nachteile auf, z.B. ein nicht befriedigendes Wasch- und Schäumvermögen, hohe Empfindlichkeit des Schaumes gegenüber Wasserhärte und gegenüber Fettbelastung. Die Herstellung von Fettsäure-Proteinhydrolysat-Kondensationsprodukten erfordert darüber hinaus entweder den Einsatz von Fettsäurechloriden oder - im Falle der Fettsäuremethylester - von hochsiedenden Lösungsmitteln (z.B. Ethylenglykol oder Dimethylsulfoxid), die im Endprodukt unerwünscht sind.

Es wurde nun eine neue Möglichkeit gefunden, Proteinhydrolysate als hydrophile Gruppe in Tenside einzubauen, wobei Produkte mit verbesserten anwendungstechnischen Eigenschaften erhalten werden. Gegenstand der Erfindung sind Oligopeptid-Derivate der allgemeinen Formel I

$$MOOC - CHR^1 - CHR^2 - CONH - R^3 \qquad ... (I)$$

in der eine der Gruppen $R^1$ oder $R^2$ Wasserstoff oder eine Alkylgruppe mit 1 - 4 C-Atomen und die andere eine Alkyl- oder Alkenylgruppe mit 6 - 22 C-Atomen darstellt, $R^3$ für den Rest eines Oligopeptids, das durch partielle Hydrolyse eines tierischen oder pflanzlichen Proteins zu einem Hydrolysat mit einem mittleren Molekulargewicht von 200 - 20 000 gewonnen wurde und gegebenenfalls am N-Atom basischer Aminosäure-Seitengruppen weitere $MOOC - CHR^1 - CHR^2 - CO$-Gruppen gebunden enthält, und M Wasserstoff oder ein Alkali-, Erdalkali-, Ammonium, Mono-, Di- oder Trialkanolammoniumion mit 1 - 4 C-Atomen in der Alkanolgruppe darstellt. Die Gruppe $R^3$ stellt den Rest eines Oligopeptids dar, das mit der freien alpha-Aminogruppe in das Amid der Alkyl- oder Alkenylbernsteinsäure überführt ist.

Die Herstellung der Oligopeptidderivate der Formel I erfolgt ausgehend von einem substituierten Bernsteinsäureanhydrid der Formel II

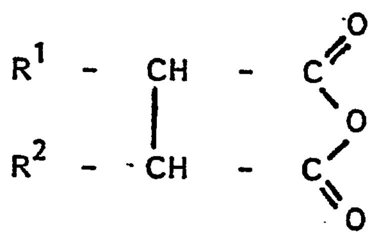

(II)

in der $R^1$ und $R^2$ die für die Formel I genannte Bedeutung haben, durch Umsetzung mit der wäßrigen Lösung eines Proteinhydrolysats der allgemeinen Formel $R^3 - NH_2$, in der $R^3$ die vorgenannte Bedeutung hat, in Gegenwart einer Base aus der Gruppe der Alkali- oder Erdalkalihydroxide, des Ammoniums oder eines Mono-, Di- oder Trialkanolamins mit 1 - 4 C-Atomen in der Alkanolgruppe bei einem pH-Wert oberhalb 8, zweckmäßigerweise in einem Bereich von pH = 8 bis pH = 12.

Alkyl- und Alkenylbernsteinsäureanhydride der Formel II sind bekannt und können z.B. auf folgende Weise erhalten werden: Nach US-PS 2.411.215 werden Alkenylbernsteinsäureanhydride aus Maleinsäureanhydrid und Monoolefinen durch En-Addition hergestellt. Als Monoolefine werden bevorzugt lineare alpha-Olefine, z.B. Petroleum-Crack-Olefine oder Ziegler-Olefine mit 6 - 22 C-Atomen eingesetzt. Es können aber auch verzweigte Monoolefine, wie sie z.B. durch Oligomerisierung von Propylen oder Butenen zugänglich sind, z.B. Tripropylen, Tetrapropylen, Diisobuten, Triisobuten und andere, verzweigte Monoolefine mit 6 - 22 C-Atomen verwendet werden.

Eine weitere Möglichkeit zur Herstellung von Alkenylbernsteinsäureanhydriden besteht darin, daß man Citraconsäureanhydrid oder Itaconsäureanhydrid mit einem Monoolefin mit 6 - 22 C-Atomen in einer En-Reaktion umsetzt. Ausgehend von Citraconsäureanhydrid erhält man auf diese Weise 1-Methyl-2-alkenyl-bernsteinsäureanhydride.

Die Alkenylbernsteinsäureanhydride können durch Hydrierung der Doppelbindung leicht in die entsprechenden Alkylbernsteinsäureanhydride überführt werden.

Die Hydrolyse von Proteinen zur Herstellung wäßriger Proteinhydrolysate mit einem Gehalt an Oligopeptiden der Formel $R^3$ - $NH_2$, mit einem mittleren Molekulargewicht von 200 - 20000 kann nach verschiedenen, an sich bekannten, Verfahren durchgeführt werden, z.B. durch saure Hydrolyse, alkalische Hydrolyse oder durch enzymatische Spaltung der in Wasser dispergierten Proteine. Die alkalische Proteinhydrolyse wird z.B. so durchgeführt, daß man eine Aufschlämmung eines Proteinpräparates mit wäßrigem Alkali bei 50 - 150°C etwa 1 - 20 Stunden behandelt, wobei die Menge des verwendeten Alkali, z.B. NaOH oder $Ca(OH)_2$ etwa 3 - 10 Gew.-%, bezogen auf das Protein, beträgt. Durch die Konzentration des Alkali, die Temperatur und die Dauer der Hydrolyse kann der Abbaugrad, also das mittlere Molekulargewicht des erhaltenen Oligopeptids gesteuert werden. Die analytische Bestimmung des Abbaugrades kann z.B. durch potentiometrische Titration des Gehaltes (a) an alpha-Aminogruppen im pH-Bereich von 6,0 - 8,5 in dem Hydrolysat, bezogen auf den Feststoffanteil (in mVal/g), erfolgen. Unter der Voraussetzung, daß ausschließlich primäre alpha-Aminogruppen erfaßt werden, läßt sich aus dem Wert (a) das mittlere Molekulargewicht des Oligopeptids

$$\left(\overline{MG} = \frac{1000}{a}\right)$$

errechnen. Da aber die im Protein enthaltenen basischen Seitengruppen der basischen Aminosäuren (Arginin, Ornithin, Lysin, Hydroxylysin, Histidin) zum geringen Teil oder ganz mittitriert werden, wird der Wert des so errechneten mittleren Molekulargewichts geringfügig unter dem realen mittleren Molekulargewicht liegen.

Die saure Proteinhydrolyse kann z.B. mit wäßriger Salzsäure oder wäßriger Schwefelsäure durchgeführt werden, wobei die Aufschlämmung des Proteins in einer 5 - 40 %igen wäßrigen Säure für einen Zeitraum von z.B. 1 - 20 Stunden unter Rückfluß zum Sieden erhitzt wird. Durch Erhitzen auf Temperaturen über 100°C unter Druck kann die Reaktion erheblich beschleunigt werden.

Der enzymatische Abbau von Proteinen wird bevorzugt unter Verwendung einer Protease durchgeführt, es können aber auch Enzympräparate verwendet werden, die z.B. Protease und Amylasen enthalten.Die enzymatische Hydrolyse macht es möglich, unter Verwendung ausgewählter proteolytischer Enzyme selektiv oder bevorzugt Bindungen zwischen ganz bestimmten Aminosäuren hydrolytisch zu spalten und auf diese Weise Oligopeptide zu erhalten, die bevorzugt ganz bestimmte Endgruppen aufweisen. So werden z.B. in Gegenwart des proteolytischen Enzyms Trypsin bevorzugt die Carbonamidgruppen des Lysins und Arginis, durch das Enzym Pepsin bevorzugt die Carbonamidgruppen von Leucin, Tyrosin, Phenylalanin und Glutaminsäure hydrolysiert.

Die enzymatische Hydrolyse kann auch mehrstufig durchgeführt werden, wobei in jeder Stufe die für das jeweils eingesetzte Enzym optimalen Bedingungen in bezug auf pH-Wert und Konzentration eingehalten werden können. Die Verfahren zur enzymatischen Proteinhydrolyse sind literaturbekannt. In Houben-Weyl, "Methoden der organischen Chemie", Band XI/2, (1958), S. 297 - 298 ist ein Verfahren zur enzymatischen Spaltung von Casein in zwei Stufen unter Verwendung von Pankreatin bei pH = 8 und mit Erepsin (Säugetierdünndarm-Präparat) bei pH = 7,6 beschrieben.

Die Proteinhydrolyse kann auch in der Weise mehrstufig erfolgen, daß z. B. zuerst eine milde saure oder alkalische Hydrolyse und danach eine enzymatische Hydrolyse zur Herstellung eines Oligopeptids des gewünschten mittleren Molekulargewichtsbereiches durchgeführt wird.

Bevorzugt geeignet zur Herstellung der erfindungsgemäßen Oligopeptid-Derivate sind Proteinhydrolysate, die bis zu einem mittleren Molekulargewicht der Oligopeptide von 400 - 4000 abgebaut sind.

Die durch die beschriebenen Hydrolyseverfahren erhaltenen wäßrigen Hydrolysate werden entweder unverdünnt oder nach Wasserzugabe zur Einstellung des geeigneten Feststoffgehalts in einem Konzentrationsbereich von 5 - 50 Gew.-% weiter umgesetzt. Geeignete Proteinpräparate können tierischer, pflanzlicher oder mikrobieller Herkunft sein. Wichtig ist, daß der Gehalt an Protein möglichst hoch, d. h. der Gehalt an Nebenprodukten wie z. B. Fetten, Kohlehydraten usw. möglichst niedrig ist. Solche geeigneten Proteine sind z. B. Kollagen aus Haut oder Bindege webe, Casein, Gelatine, Albumin aus Blut, Milch oder Eiern, Keratin aus Haaren, Wolle, Horn, Klauen und Federn, Protein aus Lederabfällen, Sojabohnenprotein, Mandelproteine und Einzellerproteine.

Die Umsetzung der substituierten Bernsteinsäureanhydride der Formel II mit der wäßrigen Lösung des Proteinhydrolysats erfolgt beispielsweise in einem Verhältnis von 1 Mol des Oligopeptids (berechnet aus dem Gehalt an freien alpha-Aminogruppen) zu 0,6 - 6 Mol des substituierten Bernsteinsäureanhydrids, bevorzugt von 1 Mol des Oligopeptids zu 1 - 3 Mol des substituierten Bernsteinsäureanhydrids. Durch Zugabe von verdünntem Alkali, z. B. von verdünnter Natronlauge wird der pH-Wert des Reaktionsgemisches auf einen Wert oberhalb 8, bevorzugt von pH = 9 bis pH = 11 gehalten. Die Reaktionstemperatur beträgt zweckmäßigerweise 50 - 80 °C. Unter diesen Bedingungen ist nach 2 - 6 Stunden kein Alkyl- oder Alkenylbernsteinsäureanhydrid mehr nachweisbar. Die Zugabe des Alkenylbernsteinsäureanhydrids kann entweder lösungsmittelfrei oder als Lösung in einem niedrigsiedenden, wassermischbaren,

aprotischen Lösungsmittel wie z. B. Aceton, Butanon, Tetrahydrofuran, 1,2 Dimethoxyethan oder Acetonitril vorgenommen werden.

Als Nebenreaktion tritt eine Hydrolyse des Alkyl- oder Alkenylbernsteinsäureanhydrids zur Alkyl- oder Alkenylbernsteinsäure ein. Durch Neutralisation des Reaktionsgemisches, z. B. mit verdünnter Mineralsäure wie Salzsäure oder Schwefelsäure auf pH = 5 - 7 und Extraktion mit einem nicht wassermischbaren Lösungsmittel kann das Reaktionsgemisch von nicht mit dem Oligopeptid verknüpften, wasserunlöslichen Nebenprodukten, in der Hauptsache also von Alkyl- oder Alkenylbernsteinsäure, befreit werden. Ein geeignetes Lösungsmittel für diese Extraktion ist z. B. Methyl-tert.butylether (MTBE). Nicht umgesetztes Proteinhydrolysat, welches ebenfalls in geringem Umfang in dem Reaktionsprodukt vorhanden ist, kann in dem Produkt verbleiben, da es die anwendungstechnischen Eigenschaften kaum stört und die kosmetisch-dermatologischen Eigenschaften der Oligopeptid-Derivate eher verbessert.

Besonders gute anwendungstechnische Eigenschaften zeigen erfindungsgemäße Oligopeptid-Derivate der Formel I, wobei eine der Gruppen $R^1$ oder $R^2$ Wasserstoff und die andere eine lineare Alkyl- oder Alkenylgruppe mit 8 - 18 C-Atomen ist. $R^3$ steht bevorzugt für den Rest eines Oligopeptides, das durch partielle Hydrolyse eines tierischen Proteins, bevorzugt einer Gelatine oder eines Lederproteins bis zu einem mittleren Molekulargewicht der Oligopeptide von 400 - 4000 gewonnen wurde.

Die erfindungsgemäßen Oligopeptid-Derivate stellen neue, gut schäumende und besonders haut- und schleimhautverträgliche Tenside dar. Diese eignen sich zur Verwendung als Tenside in flüssigen Wasch- und Reinigungsmitteln, insbesondere in kosmetischen Waschmitteln für die Haut und die Haare. Aber auch in manuellen Geschirrspülmitteln oder in Wasch- und Reinigungsmitteln, die bei der Anwendung mit der Haut in Berührung kommen, ist die Verwendung der neuen Oligopeptid-Derivate von Vorteil. Die erfindungsgemäßen Oligopeptid-Derivate sind mit allen bekannten anionischen, nichtionischen, amphoteren und zwitterionischen oberflächenaktiven Stoffen problemlos mischbar und verträglich. Sie haben den Vorteil, daß sie in Mischung mit den besonders stark schäumenden Aniontensinden, insbesondere den Alkylsulfaten und Alkylethersulfaten, deren Schäumvermögen nicht negativ beeinflussen. Sie eignen sich daher ausgezeichnet als Co-Tenside zur Verbesserung der dermatologischen Eigenschaften und zur Verbesserung der haarkosmetischen Effekte von kosmetischen Tensidzubereitungen zur Reinigung und Pflege der Haut und der Haare, z. B. Shampoos, Schaumbäder, Duschbäder, flüssige Seifen, Waschlotionen.

Solche Zubereitungen enthalten bevorzugt 3 - 30 Gew.-% Alkyl(polyglycolether)-sulfate der allgemeinen Formel III

$$R^4\text{-}0\text{-}(C_2H_40)_n\text{-}S0_3\ M^1\ \ldots\ (III)$$

in der $R^4$ eine bevorzugt lineare Alkylgruppe mit 10 - 16 C-Atomen, n = o oder eine Zahl von 1 - 12 und $M^1$ ein Alkali-, Ammonium-, Mono-, Di- oder Trialkanolammoniumion mit 1 - 4 C-Atomen in der Alkanolgruppe oder, im Falle der Alkylpolyglycolethersulfate (n = 1 - 12) auch ein Magnesiumion darstellen kann. Die Oligopeptid-Derivate sind in den kosmetischen Tensidzubereitungen bevorzugt in einem Gewichtsverhältnis von 1 : 20 bis 1 : 1 zu den Alkylsulfaten und/oder Alkylethersulfaten enthalten.

Die folgenden Beispiele sollen den Gegenstand der Erfindung näher erläutern:

Beispiele

1. Herstellung von Proteinhydrolysaten

Partielle Hydrolyse von Gelatine

1.1 600 g Gelatine wurden in 900 ml Wasser aufgenommen, mit 24 g Natriumhydroxid versetzt und 16 Stunden in einem Rührautoklaven auf 130 °C erhitzt. Anschließend wurde die Lösung durch Zugabe von 1.500 ml Wasser auf einen Gehalt von ca. 20 Gew.-% Feststoff verdünnt.
Durch potentiometrische Titration wurde der Gehalt (a) an alpha-Aminogruppen in dem Hydrolysat, bezogen auf den Feststoffanteil mVal/g bestimmt. Daraus wurde das mittlere Molekulargewicht des Oligopeptids nach

$$(\overline{MG} = \frac{1000}{a})$$

errechnet. Das mittlere Molekulargewicht ($\overline{MG}$) betrug 1060.

1.2 100 g Gelatine wurden in 150 ml Wasser aufgenommen und mit 4 g Calciumhydroxid versetzt. Der Ansatz wurde 16 Stunden auf 130 °C im Rührautoklaven erhitzt. Anschließend wurde die Lösung durch Zugabe von 250 ml Wasser auf einen Gehalt von ca. 20 Gew.-% Feststoff verdünnt. Das Proteinhydrolysat hatte ein mittleres Molekulargewicht von ca. 1690.

Partielle Hydrolyse von Ledereiweiß

1.3 400 g Lederprotein (aus Lederabfällen) wurden in 900 ml Wasser suspendiert, mit 16 g Natriumhydroxid versetzt und nach 16 Stunden im Rührautoklaven auf 130 °C erhitzt. Anschließend wurde von wenig ungelöstem Rückstand abfiltriert und durch Zugabe von 700 ml Wasser auf einen Gehalt von ca. 20 Gew.-% Feststoff verdünnt. Das Produkt hatte ein mittleres Molekulargewicht von 1030.

1.4 100 g Lederprotein (aus Lederabfällen) wurden in 150 ml Wasser suspendiert, die Suspension mit 4 g Calciumhydroxid versetzt und 16 Stunden in einem Rührautoklaven auf 130 °C erhitzt. Nach Abtrennung von wenig ungelöstem Rückstand wurde durch Zugabe von 250 ml Wasser ein Gehalt von ca. 20 Gew.-% Feststoff eingestellt. Das mittlere Molekulargewicht des Hydrolysats betrug 1970.

Herstellung von Umsetzungsprodukten aus Alkenylbernsteinsäureanhydrid und Proteinhydrolysat

2.1 100 g einer Proteinhydrolysatlösung nach 1.1 wurden bei 70 °C unter Rühren mit 10,6 g (0,04 Mol) n-Dodecenylbernsteinsäureanhydrid versetzt. Der pH-Wert des Reaktionsgemisches wurde durch Zugabe von verdünnter Natronlauge auf 11 gehalten. Nach 4 Stunden Reaktionszeit wurde der Ansatz auf 20 °C abgekühlt und mit verdünnter Salzsäure auf einen pH-Wert von 7 eingestellt.
Aus dem Reaktionsgemisch wurden durch Extraktion mit Methyl-tert.-Butylether (MTBE) 2,5 g wasserunlöslicher Nebenprodukte, (hauptsächlich nicht umgesetzte Alkenylbernsteinsäure) extrahiert. Spuren von MTBE wurden aus der wäßrigen Phase durch Erwärmen auf 40 °C unter Wasserstrahlvakuum entfernt.
Die Analyse des wasserfreien Feststoffes ergab 9,7 Gew.-% N und 2,7 Gew.-% Cl.

2.2 Nach dem Verfahren analog 2.1 wurden 100g der Proteinhydrolysatlösung nach 1.1 mit 22.6g (0,06 Mol) eines Gemisches aus n-Hexadecenyl- und n-Octadecenylbernsteinsäureanhydrid (Gewichtsverhältnis 55 : 45) bei einem pH-Wert von 10 umgesetzt. Durch Extraktion mit MTBE wurden ca. 4,7 g nicht wasserlösliche Nebenprodukte aus dem Reaktionsgemisch abgetrennt.
Die Analyse des getrockneten Feststoffs ergab 7,9 Gew.-% N, 2,4 Gew.-% Cl und 2,0 Gew.-% $H_20$.

2.3 Nach dem Verfahren analog 2.1 wurden 100 g der Proteinhydrolysat-Lösung nach 1,3 mit 21,3 g (0,08 Mol) eines n-Dodecenylbernsteinsäureanhydrids umgesetzt. Durch Extraktion mit MTBE wurden ca. 7 g nicht wasserlösliche Nebenprodukte aus dem Reaktionsgemisch abgetrennt.
Die Analyse des getrockneten Feststoffs ergab 10,1 Gew.-% N, 2,8 Gew.-% Cl.

2.4 Nach dem Verfahren analog 2.1 wurden 100 g der Proteinhydrolysat-Lösung nach 1.3 mit 31,9 g (0,12 Mol) eines n-Dodecenylbernsteinsäureanhydrids umgesetzt. Durch Extraktion mit MTBE wurden aus dem Reaktionsgemisch 12,8 g nicht wasserlösliche Nebenprodukte abgetrennt.
Die Analyse des getrockneten Feststoffs ergab 11,1 Gew.-% N.

2.5 Nach dem Verfahren analog 2.1 wurden 100 g der Proteinhydrolysatlösung nach 1.1 mit 22,6 g (0,10 Mol) eines n-Dodecenylbernsteinsäureanhydrids bei 50 °C und einem pH-Wert von 10,5 umgesetzt. Aus dem Reaktionsgemisch wurden mit MTBE 16,8 g nicht wasserlösliche Nebenprodukte abgetrennt.

2.6 Nach dem Verfahren analog 2.1 wurden 100 g der Proteinhydrolysat-Lösung nch 1.2 mit 16 g (0,06 Mol) eines n-Dodecenylbernsteinsäureanhydrids umgesetzt. Die Neutralisation wurde mit verdünnter Schwefelsäure durchgeführt. Von geringen Mengen ungelöster Anteile wurde abfiltriert. Durch Extraktion mit MTBE wurden 6,4 g nicht wasserlösliche Nebenprodukte aus dem Ansatz abgetrennt. Die Analyse des getrockneten Produktes ergab 13,9 Gew.-% N.

2.7 Nach dem Verfahren analog 2.1 wurden 100 g der Proteinhydrolysat-Lösung nach 1.4 mit 16 g (0,06 Mol) n-Dodecenylbernsteinsäureanhydrid umgesetzt. Durch Extraktion mit MTBE wurden 4,2 g nicht wasserlösliche Nebenprodukte aus dem Ansatz abgetrennt.
Die Analyse des getrockneten Umsetzungsproduktes ergab: 12,6 Gew.-% N.

Prüfung der Schäumeigenschaften.

Das Schäumvermögen wurde geprüft nach DIN 53902-T01. Das Prüfverfahren besteht darin, daß durch 30 Sekunden langes Schlagen von 200 ml einer Lösung des Tensids in einem 1 l-Standzylinder mit einer an einem Stiel befestigten Lochplatte mit 1 Schlag pro Sekunde Schaum erzeugt wird. Das Schaumvolumen der Probelösung wird 30 Sekunden nach Beendigung des Schlagens gemessen.

In der Tabelle I ist das Schaumvolumen bei 40 °C für Produktkonzentrationen von 0,5 g/l, 1,0 g/l, 2,0 g/l und 3 g/l bei einer Wasserhärte von 0 °d., 10 °d. und 20 °d angegeben.

In Tabelle II ist das Schaumvolumen für eine Mischung aus Fettalkohol ($C_{12}/C_{14}$)-poly (2EO)glycolethersulfat, Na-Salz (FES) und dem Oligopeptid-Derivat im Gewichtsverhältnis 7 : 3 bei 40 °C und bei abgestuften Gesamt-Konzentrationen von 0,5 g/l, 1,0 g/l, 2,0 g/l und 3 g/l bei einer Wasserhärte von 10 °d.H. angegeben (1 °d entspricht 10 mg/l Ca0 oder 0,357 mval/l Erdalkaliionen).

Tabelle I

| Oligopeptid-Derivat | Konzentration | Wasserhärte 0 °d | 10 °d | 20 °d |
|---|---|---|---|---|
| Beispiel 2.3 | 0,5 g/l | 100 ml | 130 ml | 160 ml |
| | 1,0 g/l | 130 ml | 230 ml | 250 ml |
| | 2,0 g/l | 250 ml | 350 ml | 390 ml |
| | 3,0 g/l | 500 ml | 520 ml | 500 ml |
| Beispiel 2.7 | 0,5 g/l | 240 ml | 70 ml | 50 ml |
| | 1,0 g/l | 250 ml | 110 ml | 160 ml |
| | 2,0 g/l | 390 ml | 190 ml | 210 ml |
| | 3,0 g/l | 560 ml | 240 ml | 240 ml |

Tabelle II

| Tensid | Konzentration | Schaumvolumen bei 10 °d |
|---|---|---|
| FES : 70 Gew.% Beispiel 2,3 : 30 Gew.% | 0,5 g/l | 500 ml |
| | 1,0 g/l | 670 ml |
| | 2,0 g/l | 790 ml |
| | 3,0 g/l | 900 ml |
| FES | 1,0 g/l | 740 ml |
| | 3,0 g/l | 910 ml |

**Patentansprüche**

1. Oligopeptid-Derivate der allgemeinen Formel I

$$MOOC\text{-}CHR^1\text{-}CHR^2\text{-}CONH\text{-}R^3 \quad ... \text{(I)}$$

in der eine der Gruppen $R^1$ oder $R^2$ Wasserstoff oder eine Alkylgruppe mit 1 - 4 C-Atomen und die andere eine Alkyl- oder Alkenylgruppe mit 6 - 22 C-Atomen darstellt, $R^3$ für den Rest eines Oligopeptids, das durch partielle Hydrolyse eines tierischen oder pflanzlichen Proteins zu einem Hydrolysat mit einem mittleren Molekulargewicht von 200 - 20 000 gewonnen wurde und gegebenenfalls am N-Atom basischer Aminosäure-Seitengruppen weitere MOOC - $CHR^1$ - $CHR^2$ - CO-Gruppen gebunden enthält, und M Wasserstoff oder ein Alkali-, Erdalkali-, Ammonium, Mono-, Di- oder Trialkanolammoniumion mit 1 - 4 C-Atomen in der Alkanolgruppe darstellt.

2. Oligopeptid-Derivate nach Anspruch 1, dadurch gekennzeichnet, daß eine der Gruppen $R^1$ oder $R^2$ Wasserstoff und die andere eine lineare Alkyl- oder Alkenylgruppe mit 8 - 18 C-Atomen darstellt.

3. Oligopeptid-Derivat nach Anspruch 1 oder 2, dadurch gekennzeichnet, daß das Oligopeptid durch partielle Hydrolyse eines tierischen Proteins, bevorzugt von Gelatine oder Lederprotein zu einem Hydrolysat mit einem mittleren Molekulargewicht von 400 - 4 000 gewonnen wurde.

4. Verfahren zur Herstellung von Oligopeptid-Derivaten der allgemeinen Formel I

$$MOOC\text{-}CHR^1\text{-}CHR^2\text{-}CONH\text{-}R^3 \qquad (I)$$

in der eine der Gruppen $R^1$ oder $R^2$ Wasserstoff oder eine Alkylgruppe mit 6 - 22 C-Atomen darstellt und $R^3$ für den Rest eines Oligopeptids, das durch partielle Hydrolyse eines tierischen oder pflanzlichen Proteins zu einem Hydrolysat mit einem mittleren Molekulargewicht von 200 - 20 000 gewonnen wurde und gegebenenfalls am N-Atom basischer Aminosäure-Seitengruppen weitere MOOC - $CHR^1$ - $CHR^2$ - CO-Gruppen gebunden enthält, und M ein Alkali-, Erdalkali-, Ammonium-, Mono-, Di- oder Trialkanolammonium-Kation mit 1 - 4 C-Atomen in der Alkanolgruppe darstellt, dadurch gekennzeichnet, daß man ein substituiertes Bernsteinsäureanhydrid der allgemeinen Formel II

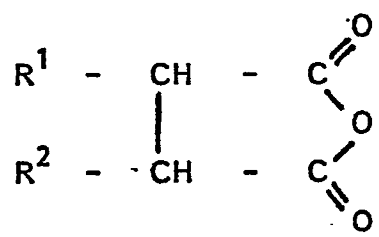

(II)

in der $R^1$ und $R^2$ die vorgenannte Bedeutung haben, mit einer wäßrigen Lösung eines Proteinhydrolysats der allgemeinen Formel $H_2N\text{-}R^3$, in der $R^3$ die vorgenannte Bedeutung hat, in Gegenwart einer Base aus der Gruppe der Alkali- oder Erdalkalihydroxide, des Ammoniums oder eines Mono-, Di- oder Trialkanolamins mit 1 - 4 C-Atomen in der Alkanolgruppe bei einem pH-Wert oberhalb 8 zur Umsetzung bringt.

5. Verfahren nach Anspruch 4, dadurch gekennzeichnet, daß das Reaktionsgemisch durch Extraktion mit einem organischen, nicht wassermischbaren Lösungsmittel bei einem pH-Wert von 5 - 7 von nicht mit dem Oligopeptidrest verknüpften wasserunlöslichen Nebenprodukten befreit wird.

6. Verwendung der Oligopeptid-Derivate nach Anspruch 1 - 3 als hautfreundliche Tenside in flüssigen Wasch- und Reinigungsmitteln, insbesondere in kosmetischen Waschmitteln für die Haut und die Haare.

**Claims**

1. Oligopeptide derivatives corresponding to the following general formula

$$MOOC\text{–}CHR^1\text{–}CHR^2\text{–}CONH\text{–}R^3 \qquad (I)$$

in which one of the groups $R^1$ or $R^2$ represents hydrogen or a $C_1$–$C_4$ alkyl group whilst the other represents a $C_6$–$C_{22}$ alkyl or alkenyl group, $R^3$ represents the residue of an oligopeptide which has been obtained by partial hydrolysis of an animal or vegetable protein to a hydrolyzate having an average molecular weight of from 200 to 20,000 and which optionally contains other MOOC–$CHR^1$–$CHR^2$–CO-groups attached to the nitrogen atom of basic amino acid side groups, and M represents hydrogen or an alkali, alkaline earth, ammonium, mono-, di- or trialkanolammonium ion containing from 1 to 4 carbon atoms in the alkanol group.

2. Oligopeptide derivatives as claimed in Claim 1, characterized in that one of the groups $R^1$ or $R^2$ represents hydrogen whilst the other is a linear $C_{8-18}$ alkyl or alkenyl group.

3. An oligopeptide derivative as claimed in Claim 1 or 2, characterized in that the oligopeptide has been obtained by partial hydrolysis of an animal protein, preferably gelatin or leather protein, to a hydrolyzate having an average molecular weight of from 400 to 4000.

4. A process for the production of oligopeptide derivatives corresponding to the following general formula

$$MOOC\text{–}CHR^1\text{–}CHR^2\text{–}CONH\text{–}R^3 \qquad (I)$$

in which one of the groups $R^1$ or $R^2$ represents hydrogen or a $C_6$–$C_{22}$ alkyl group and $R^3$ represents the residue of an oligopeptide which has been obtained by partial hydrolysis of an animal or vegetable protein to a hydrolyzate having an average molecular weight of from 200 to 20,000 and which optionally contains other $MOOC–CHR^1–CHR^2–CO$-groups attached to the nitrogen atom of basic amino acid side groups, and M represents an alkali, alkaline-earth, ammonium, mono-, di- or trialkanolammonium cation containing from 1 to 4 carbon atoms in the alkanol group, characterized in that a substituted succinic acid anhydride corresponding to the following general formula

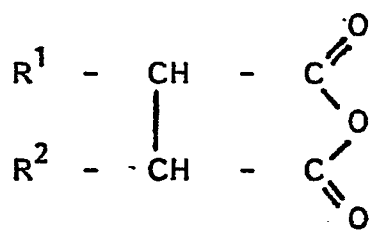

(II)

in which $R^1$ and $R^2$ are as defined above, is reacted with an aqueous solution of a protein hydrolyzate corresponding to the general formula $H_2N–R^3$, where $R^3$ is as defined above, in the presence of a base from the group comprising alkali or alkaline-earth hydroxides, ammonium or a mono-, di- or trialkanolamine containing from 1 to 4 carbon atoms in the alkanol group at a pH value above 8.

5. A process as claimed in Claim 4, characterized in that water-insoluble secondary products which are not attached to the oligopeptide residue are removed from the reaction mixture by extraction with an organic water-immiscible solvent at a pH value of from 5 to 7.

6. The use of the oligopeptide derivatives claimed in Claims 1 to 3 as surfactants kind to the skin in liquid washing and cleaning preparations, particularly in cosmetic washing preparations for the skin and the hair.

## Revendications

1. Dérivés d'oligopeptides de formule générale I

$$MOOC–CHR^1–CHR^2–CONH–R^3 \quad (I)$$

dans laquelle un des groupements $R^1$ ou $R^2$ représente l'hydrogène ou un groupement alkyle comportant 1 à 4 atomes de C et l'autre un groupement alkyle ou alcényle comprenant 6 à 22 atomes de C, $R^3$ représente le résidu d'un oligopeptide, obtenu par hydrolyse partielle d'une protéine animale ou végétale en un hydrolysat de poids moléculaire moyen allant de 200 à 20 000, et qui comporte le cas échéant d'autres groupements $MOOC–CHR^1–CHR^2–CO$ fixés sur l'atome N des groupements latéraux basiques d'acides aminés, et M représente l'hydrogène ou un ion de métal alcalin, de métal alcalino-terreux, d'ammonium, de mono-, de di- ou de trialcanolammonium, dont le groupement alcanol comporte 1 à 4 atomes de C.

2. Dérivés d'oligopeptides selon la revendication 1, caractérisés en ce que l'un des groupements $R^1$ ou $R^2$ représente l'hydrogène et l'autre un groupement alkyle ou alcényle comportant 8 à 18 atomes de C.

3. Dérivés d'oligopeptides selon la revendication 1 ou 2, caractérisés en ce que l'oligopeptide a été obtenu par hydrolyse partielle d'une protéine animale, de préférence la gélatine ou une protéine de cuir en un hydrolysat de poids moléculaire moyen allant de 400 à 4000.

4. Procédé de préparation de dérivés d'oligopeptides de formule générale I

$$MOOC–CHR^1–CHR^2–CONH–R^3 \quad (I)$$

dans laquelle un des groupements $R^1$ ou $R^2$ représente l'hydrogène ou un groupement alkyle comportant 6 à 22 atomes de C et $R^3$ le résidu d'un oligopeptide, obtenu par hydrolyse partielle d'une protéine animale ou végétale en un hydrolysat de poids moléculaire moyen allant de 200 à 20 000, et qui comporte le cas échéant d'autres groupements $MOOC–CHR^1–CHR^2–CO$ fixés sur l'atome N des groupements latéraux basiques d'acides aminés, et M représente un cation de métal alcalin, de métal alcalino-terreux, d'ammonium, de mono-, de di- ou de trialcanolammonium, dont le groupement alcanol comporte 1 à 4 atomes de C, caractérisé en ce que l'on fait réagir à un pH supérieur à 8 un anhydride d'acide succinique substitué de formule générale II

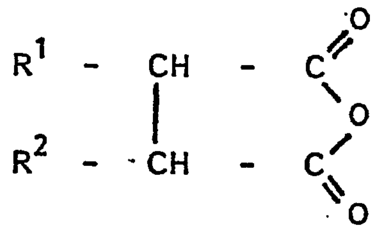

(II)

dans laquelle $R^1$ et $R^2$ possèdent la signification précitée, avec une solution aqueuse d'un hydrolysat de protéines de formule générale $H_2N-R^3$ dans laquelle $R^3$ possède la signification précitée, en présence d'une base appartenant au groupe des hydroxydes de métaux alcalins ou de métaux alcalino-terreux, de l'ammonium ou d'une mono, di-, ou trialcanolamine dont le groupement alcanol comporte 1 à 4 atomes de C.

5. Procédé selon la revendication 4 caractérisé en ce que le mélange réactionnel est débarrassé des sous-produits insolubles dans l'eau non liés au résidu d'oligopeptide par extraction à un pH de 5 à 7 au moyen d'un solvant organique et non miscible avec l'eau.

6. Utilisation des dérivés d'oligopeptides selon les revendications 1 à 3 comme tensioactifs bien tolérés par la peau dans des produits liquides de lavage et de nettoyage, en particulier dans des produits de lavage cosmétiques destinés à la peau et à la chevelure.